(19) 

Europäisches
Patentamt
European
Patent Office
Office européen
des brevets

(11) **EP 4 098 238 A1**

(12) **EUROPEAN PATENT APPLICATION**
published in accordance with Art. 153(4) EPC

(43) Date of publication:
**07.12.2022 Bulletin 2022/49**

(21) Application number: **21747929.4**

(22) Date of filing: **25.01.2021**

(51) International Patent Classification (IPC):
**A61J 1/05** (2006.01)

(52) Cooperative Patent Classification (CPC):
**A61J 1/05**

(86) International application number:
**PCT/JP2021/002477**

(87) International publication number:
**WO 2021/153511 (05.08.2021 Gazette 2021/31)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **28.01.2020 JP 2020011941**

(71) Applicant: **Zeon Corporation
Tokyo 100-8246 (JP)**

(72) Inventor: **SAWAGUCHI Taichi
Tokyo 100-8246 (JP)**

(74) Representative: **Pfenning, Meinig & Partner mbB
Patent- und Rechtsanwälte
Theresienhöhe 11a
80339 München (DE)**

(54) **PREFILLED DRUG PACKAGE AND PRODUCTION METHOD FOR PREFILLED DRUG PACKAGE**

(57)     Provided is a pre-filled drug package that can inhibit protein aggregation after long-term storage even when accommodating a protein solution formulation having a low non-ionic surfactant concentration. The pre-filled drug package includes a protein solution formulation and a housing accommodating the protein solution formulation. The protein solution formulation has a non-ionic surfactant concentration of not less than 0 mg/mL and less than 0.05 mg/mL. At least part of a section of the housing that is in contact with the protein solution formulation is formed of a resin including either or both of a hydrogenated cycloolefin ring-opened polymer and a copolymer of a cycloolefin and a chain olefin.

*FIG. 1*

**Description**

TECHNICAL FIELD

The present disclosure relates to a pre-filled drug package and a method of producing a pre-filled drug package.

BACKGROUND

Pre-filled drug packages that are filled with drugs in advance have been increasingly used in recent years from viewpoints of convenience during use and the ability to prevent medical accidents.

The drug with which a pre-filled drug package is filled may, for example, be a formulation that contains a protein in an aqueous solution (i.e., a protein solution formulation). A problem faced by pre-filled drug packages that are filled with protein solution formulations such as described above is that protein aggregation may occur during long-term storage.

In order to inhibit such protein aggregation, non-ionic surfactants such as polysorbate 80 and polysorbate 20 are conventionally added to protein solution formulations (for example, refer to Patent Literature (PTL) 1).

CITATION LIST

Patent Literature

PTL 1: JP2018-537170A

SUMMARY

(Technical Problem)

The inventor focused on the fact that non-ionic surfactants such as polysorbate 80 and polysorbate 20 have drawbacks in terms that they are carcinogenic and may lead to problems such as hypersensitivity and chromosome abnormalities upon administration to a human body. This is presumed to be due to decomposition products produced through decomposition of such non-ionic surfactants. For this reason, the inventor attempted to reduce the non-ionic surfactant concentration in a protein solution formulation when producing a pre-filled drug package using the conventional technique described above. However, studies carried out by the inventor revealed that when the non-ionic surfactant concentration in a protein solution formulation is reduced in a conventional pre-filled drug package such as described above, it is not possible to inhibit protein aggregation during long-term storage of the pre-filled drug package.

Accordingly, one object of the present disclosure is to provide a pre-filled drug package that can inhibit protein aggregation after long-term storage even when the pre-filled drug package accommodates a protein solution formulation having a low non-ionic surfactant concentration.

Another object of the present disclosure is to provide a method of producing a pre-filled drug package that can inhibit protein aggregation after long-term storage even when the pre-filled drug package accommodates a protein solution formulation having a low non-ionic surfactant concentration.

(Solution to Problem)

The inventor conducted diligent studies with the aim of solving the problem set forth above. The inventor discovered that with regards a housing of a pre-filled drug package accommodating a protein solution formulation, by using a specific resin to form at least part of a section of the housing that is in contact with the protein solution formulation, it is possible to reduce the tendency of protein aggregation to occur after long-term storage even in a case in which the non-ionic surfactant concentration in the protein solution formulation is substantially reduced. In this manner, the inventor completed the present disclosure.

Specifically, the present disclosure aims to advantageously solve the problem set forth above, and a presently disclosed pre-filled drug package comprises: a protein solution formulation; and a housing accommodating the protein solution formulation, wherein the protein solution formulation has a non-ionic surfactant concentration of not less than 0 mg/mL and less than 0.05 mg/mL, and at least part of a section of the housing that is in contact with the protein solution formulation is formed of a resin containing either or both of a hydrogenated cycloolefin ring-opened polymer and a copolymer of a cycloolefin and a chain olefin. Even in a situation in which the protein solution formulation has a low non-ionic surfactant concentration of not less than 0 mg/mL and less than 0.05 mg/mL in this manner, by adopting a pre-filled drug package including a housing for which at least part of a section thereof that is in contact with the protein solution formulation (hereinafter, also referred to as a "formulation contacting section") is formed of the resin set forth above, it is possible to inhibit protein aggregation in the protein solution formulation even upon long-term storage of the pre-filled drug package.

In the presently disclosed pre-filled drug package, the section of the housing that is in contact with the protein solution formulation preferably has a water contact angle of 90° or more. When the water contact angle of the formulation contacting section is 90° or more, protein aggregation in the protein solution formulation can be further inhibited even during long-term storage of the pre-filled drug package.

Note that the "water contact angle" of a formulation contacting section referred to in the present disclosure can be measured by a method described in the EXAMPLES section of the present specification.

The presently disclosed pre-filled drug package can, for example, have a configuration in which it further comprises a sealing member and in which the housing has an opening and the opening is hermetically sealed by the sealing member.

In the presently disclosed pre-filled drug package, the protein solution formulation can contain either or both of an antibody and an antigen binding fragment of the antibody.

Moreover, in the presently disclosed pre-filled drug package, the antibody can be at least one selected from the group consisting of chimeric antibodies, human antibodies, humanized antibodies, and domain antibodies of any thereof.

Furthermore, in the presently disclosed pre-filled drug package, the protein solution formulation can contain at least one selected from the group consisting of ofatumumab, cetuximab, tocilizumab, bevacizumab, canakinumab, golimumab, ustekinumab, eculizumab, omalizumab, trastuzumab, pertuzumab, adalimumab, denosumab, mogamulizumab, rituximab, ranibizumab, infliximab, aflibercept, abatacept, etanercept, gemtuzumab ozogamicin, panitumumab, basiliximab, certolizumab pegol, and palivizumab.

The presently disclosed pre-filled drug package is, for example, a vial, an infusion bag, a pre-filled cartridge, an ampoule, a bottle, a pouch, or a blister pack.

Moreover, the present disclosure aims to advantageously solve the problem set forth above, and a presently disclosed method of producing a pre-filled drug package is a method of producing a pre-filled drug package including a protein solution formulation and a housing accommodating the protein solution formulation, comprising a step of filling the protein solution formulation, with a non-ionic surfactant concentration of not less than 0 mg/mL and less than 0.05 mg/mL, into the housing to obtain the pre-filled drug package, wherein at least part of a section of the housing that is in contact with the protein solution formulation is formed of a resin containing either or both of a hydrogenated cycloolefin ring-opened polymer and a copolymer of a cycloolefin and a chain olefin. Even in a situation in which the protein solution formulation has a low non-ionic surfactant concentration of not less than 0 mg/mL and less than 0.05 mg/mL in this manner, by filling the protein solution formulation into a housing for which at least part of a section thereof that is in contact with the protein solution formulation is formed of the resin set forth, it is possible to obtain a pre-filled drug package that can inhibit protein aggregation in the protein solution formulation even upon long-term storage.

The presently disclosed method of producing a pre-filled drug package preferably further comprises, in advance of the step of obtaining the pre-filled drug package: a step of pre-drying the resin; and a step of shaping the resin after the pre-drying to obtain the housing. By using a housing that is obtained through shaping of a resin that has been pre-dried, protein aggregation in the protein solution formulation can be further inhibited during long-term storage of the pre-filled drug package that is obtained.

In the presently disclosed method of producing a pre-filled drug package, the resin preferably has an oxygen concentration of 10 mass ppm or less after the pre-drying. When the oxygen concentration in the pre-dried resin is 10 mass ppm or less, protein aggregation in the protein solution formulation can be more sufficiently inhibited during long-term storage of the pre-filled drug package that is obtained.

Note that the "oxygen concentration" in a resin referred to in the present disclosure can be measured by a method described in the EXAMPLES section of the present specification.

In the presently disclosed method of producing a pre-filled drug package, the pre-drying is preferably performed in an inert gas atmosphere. By performing pre-drying of the resin in an inert gas atmosphere, protein aggregation in the protein solution formulation can be more sufficiently inhibited during long-term storage of the pre-filled drug package that is obtained.

In the presently disclosed method of producing a pre-filled drug package, the pre-drying preferably has a drying temperature of not lower than 80°C and not higher than 120°C. By performing pre-drying of the resin at a temperature within the range set forth above, protein aggregation in the protein solution formulation can be more sufficiently inhibited during long-term storage of the pre-filled drug package that is obtained.

In the presently disclosed method of producing a pre-filled drug package, the protein solution formulation can contain either or both of an antibody and an antigen binding fragment of the antibody.

Moreover, in the presently disclosed method of producing a pre-filled drug package, the antibody can be at least one selected from the group consisting of chimeric antibodies, human antibodies, humanized antibodies, and domain antibodies of any thereof.

Furthermore, in the presently disclosed method of producing a pre-filled drug package, the protein solution formulation can contain at least one selected from the group consisting of ofatumumab, cetuximab, tocilizumab, bevacizumab, canakinumab, golimumab, ustekinumab, eculizumab, omalizumab, trastuzumab, pertuzumab, adalimumab, denosumab, mogamulizumab, rituximab, ranibizumab, infliximab, aflibercept, abatacept, etanercept, gemtuzumab ozogamicin, panitumumab, basiliximab, certolizumab pegol, and palivizumab.

The presently disclosed method of producing a pre-filled drug package is, for example, a method of producing a vial, an infusion bag, a pre-filled cartridge, an ampoule, a bottle, a pouch, or a blister pack.

(Advantageous Effect)

According to the present disclosure, it is possible to provide a pre-filled drug package that can inhibit protein aggregation after long-term storage even when the pre-filled drug package accommodates a protein solution formulation having a low non-ionic surfactant concentration.

Moreover, according to the present disclosure, it is possible to provide a method of producing a pre-filled drug package that can inhibit protein aggregation after long-term storage even when the pre-filled drug package accommodates a protein solution formulation having a low non-ionic surfactant concentration.

BRIEF DESCRIPTION OF THE DRAWINGS

In the accompanying drawings:

FIG. 1 illustrates one example of schematic configuration of a vial that is a pre-filled drug package in accordance with the present disclosure; and
FIG. 2 illustrates one example of schematic configuration of an infusion bag that is a pre-filled drug package in accordance with the present disclosure.

DETAILED DESCRIPTION

The following provides a detailed description of embodiments of the present disclosure.

The presently disclosed pre-filled drug package accommodates a protein solution formulation in a housing. Moreover, the presently disclosed pre-filled drug package can be produced by the presently disclosed method of producing a pre-filled drug package, for example.

(Pre-filled drug package)

The presently disclosed pre-filled drug package includes at least: a housing having an internal space that can accommodate a liquid such as a protein solution formulation; and a protein solution formulation that is accommodated in the internal space of the housing. Note that the presently disclosed pre-filled drug package may include constituents other than the aforementioned housing and protein solution formulation. For example, the presently disclosed pre-filled drug package may include a member (sealing member) that can seal the internal space of the housing. In a case in which the housing has an opening that connects the internal space with outside of the housing, by hermetically sealing the opening through a sealing member, it is possible to accommodate (hermetically seal) the protein solution formulation in the internal space defined by the sealing member and the housing such that it is shielded from external air while also enabling simple removal of the protein solution formulation.

The presently disclosed pre-filled drug package is not specifically limited so long as it is obtained by filling a protein solution formulation into a vessel, packaging body, or the like that can accommodate the protein solution formulation before the protein solution formulation is used, such as through administration to a human body. Specific examples of the presently disclosed pre-filled drug package include a vial, an infusion bag, a pre-filled cartridge, an ampoule, a bottle, a pouch, a blister pack, and a pre-filled syringe.

In one aspect of the present disclosure, "pre-filled syringe" can be excluded from the definition of the term "pre-filled drug package". In this aspect, "pre-filled drug package" can, for example, be denoted as "pre-filled drug package (exclusive of pre-filled syringe)", for example.

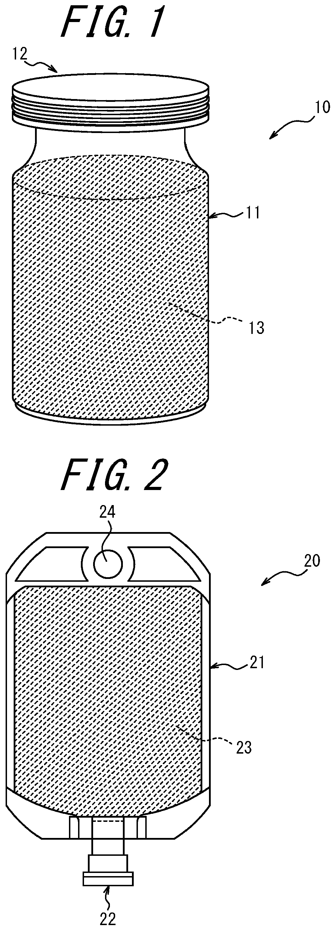

Examples of the structure of the presently disclosed pre-filled drug package are further described below using FIG. 1 and FIG. 2. Structures are described using a vial as an example of the pre-filled drug package in FIG. 1 and using an infusion bag as an example of the pre-filled drug package in FIG. 2, but the presently disclosed pre-filled drug package is not limited thereto.

<Vial>

FIG. 1 illustrates one example of a vial serving as the presently disclosed pre-filled drug package. A vial 10 illustrated in FIG. 1 includes a vessel 11 as a housing, a cap 12 as a sealing member, and a protein solution formulation 13. The vessel 11 has a roughly circular tube shape and has an opening at one end of the circular tube (upper end in FIG. 1). This opening is hermetically sealed by the cap 12. The protein solution formulation 13 is accommodated in an internal space that is defined by the vessel 11 and the cap 12. The protein solution formulation 13 can be externally removed by piercing the cap 12 with an injection needle, for example.

<Infusion bag>

FIG. 2 illustrates one example of an infusion bag serving as the presently disclosed pre-filled drug package. An infusion bag 20 illustrated in FIG. 2 includes a packaging body 21 as a housing, a port 22 as a sealing member, and a protein solution formulation 23. The packaging body 21 has a roughly rectangular shape in plan view and has an opening at one side of the rectangle (lower side in FIG. 2). This opening is hermetically sealed by the port 22. Note that a hole 24 is formed in the packaging body 21, thereby enabling hanging of the infusion bag 20 using a hook or the like.
The protein solution formulation 23 is accommodated in an internal space that is defined by the packaging body 21 and the port 22. The protein solution formulation 23 can be externally removed by piercing the port 22 with an injection needle, for example.
Features of the presently disclosed pre-filled drug package exemplified in FIG. 1 and FIG. 2, described above, are that the protein solution formulation accommodated in the housing has a non-ionic surfactant concentration of not less than 0 mg/mL and less than 0.05 mg/mL and that at least part of a section of the housing that is in contact with the protein solution formulation is obtained through shaping of a resin containing a hydrogenated cycloolefin ring-opened polymer and/or a copolymer of a cycloolefin and a chain olefin.
As a result of at least part of the housing being formed using the specific resin set forth above and as a result of the surface of a section that is formed using the specific resin being in contact with the protein solution formulation, the presently disclosed pre-filled drug package can inhibit protein aggregation after long-term storage even when the protein solution formulation has a non-ionic surfactant concentration of not less than 0 mg/mL and less than 0.05 mg/mL. Although it is not clear why such an effect is obtained, the reason is presumed to be as follows. According to the inventor, protein aggregation in a pre-filled drug package may be caused by adsorption of a protein onto an inner wall surface of a housing of the pre-filled drug package, leading to assembly of adsorbed protein. However, by forming a section of the housing of the pre-filled drug package that is in contact with the protein solution formulation using a hydrogenated cycloolefin ring-opened polymer and/or a copolymer of a cycloolefin and a chain olefin, affinity between an inner wall surface of the housing and the protein can be reduced, and thus adsorption of the protein can be prevented. It is presumed that by inhibiting adsorption of the protein to the inner wall surface in this manner, protein aggregation inside the pre-filled drug package can be inhibited.
The following further describes the protein solution formulation that is accommodated in the presently disclosed pre-filled drug package, the housing that is included in the presently disclosed pre-filled drug package, and the sealing member that is optionally included in the presently disclosed pre-filled drug package.

<Protein solution formulation>

The protein solution formulation contains at least a protein and water and has a non-ionic surfactant concentration of not less than 0 mg/mL and less than 0.05 mg/mL.

<<Protein>>

The protein contained in the protein solution formulation is not specifically limited and may, for example, be an antibody (chimeric antibody, human antibody, humanized antibody, or domain antibody of any thereof) or an antigen binding fragment of the antibody.
More specific examples of the protein include ofatumumab (product name: Arzerra® (Arzerra is a registered trademark in Japan, other countries, or both)), cetuximab (product name: Erbitux® (Erbitux is a registered trademark in Japan, other countries, or both)), tocilizumab (product name: Actemra® (Actemra is a registered trademark in Japan, other countries, or both)), bevacizumab (product name: Avastin® (Avastin is a registered trademark in Japan, other countries, or both)), canakinumab (product name: Ilaris® (Ilaris is a registered trademark in Japan, other countries, or both)), golimumab (product name: Simponi® (Simponi is a registered trademark in Japan, other countries, or both)), ustekinumab (product name: Stelara® (Stelara is a registered trademark in Japan, other countries, or both)), eculizumab (product name: Soliris® (Soliris is a registered trademark in Japan, other countries, or both)), omalizumab (product name:

Xolair® (Xolair is a registered trademark in Japan, other countries, or both)), trastuzumab (product name: Herceptin® (Herceptin is a registered trademark in Japan, other countries, or both)), pertuzumab (product name: Perjeta® (Perjeta is a registered trademark in Japan, other countries, or both)), adalimumab (product name: Humira® (Humira is a registered trademark in Japan, other countries, or both)), denosumab (product name: Prolia® (Prolia is a registered trademark in Japan, other countries, or both); product name: Ranmark® (Ranmark is a registered trademark in Japan, other countries, or both)), mogamulizumab (product name: Poteligeo® (Poteligeo is a registered trademark in Japan, other countries, or both)), rituximab (product name: Rituxan® (Rituxan is a registered trademark in Japan, other countries, or both)), ranibizumab (product name: Lucentis® (Lucentis is a registered trademark in Japan, other countries, or both)), infliximab (product name: Remicade® (Remicade is a registered trademark in Japan, other countries, or both)), aflibercept (product name: Eylea® (Eylea is a registered trademark in Japan, other countries, or both)), abatacept (product name: Orencia® (Orencia is a registered trademark in Japan, other countries, or both)), etanercept (product name: Enbrel® (Enbrel is a registered trademark in Japan, other countries, or both)), gemtuzumab ozogamicin (product name: Mylotarg® (Mylotarg is a registered trademark in Japan, other countries, or both)), panitumumab (product name: Vectibix® (Vectibix is a registered trademark in Japan, other countries, or both)), basiliximab (product name: Simulect® (Simulect is a registered trademark in Japan, other countries, or both)), certolizumab pegol (product name: Cimzia® (Cimzia is a registered trademark in Japan, other countries, or both)), and palivizumab (product name: Synagis® (Synagis is a registered trademark in Japan, other countries, or both)).

Note that the protein solution formulation may contain one type of protein or may contain two or more types of proteins. In other words, the protein solution formulation may contain both an antibody and an antigen binding fragment, may contain two or more types of antibodies, or may contain two or more types of antigen binding fragments, for example.

The concentration of the protein in the protein solution formulation is preferably 0.005 mg/mL or more, more preferably 0.01 mg/mL or more, and even more preferably 0.05 mg/mL or more, and is preferably 500 mg/mL or less, more preferably 300 mg/mL or less, and even more preferably 200 mg/mL or less. When the concentration of the protein in the protein solution formulation is 0.005 mg/mL or more, the expected effect of the protein can be sufficiently obtained when the protein solution formulation is administered to a human body or the like. Moreover, when the concentration of the protein in the protein solution formulation is 500 mg/mL or less, aggregation of the protein in the protein solution formulation can be sufficiently inhibited during long-term storage of the pre-filled drug package.

<<Non-ionic surfactant>>

A non-ionic surfactant that is optionally contained in the protein solution formulation is a component that can function as a stabilizer for stabilizing the protein described above. Examples of such non-ionic surfactants include, but are not specifically limited to, sorbitan fatty acid esters, glycerin fatty acid esters, polyglycerin fatty acid esters, polyoxyethylene sorbitan fatty acid esters (polyoxyethylene sorbitan oleate (polysorbate 80), polyoxyethylene sorbitan monolaurate (polysorbate 20), etc.), polyoxyethylene sorbitol fatty acid esters, polyoxyethylene glycerin fatty acid esters, polyoxyethylene fatty acid esters, polyoxyethylene alkyl ethers, polyoxyethylene polyoxypropylene alkyl ethers, polyoxyethylene alkyl phenyl ethers, polyoxyethylene hydrogenated castor oil, polyoxyethylene beeswax derivatives, polyoxyethylene lanolin derivatives, and polyoxyethylene fatty acid amides.

Note that one non-ionic surfactant may be used individually, or two or more non-ionic surfactants may be used in combination.

A protein solution formulation having a non-ionic surfactant concentration of not less than 0 mg/mL and less than 0.05 mg/mL is used in the presently disclosed pre-filled drug package from a viewpoint of reducing the risk of hypersensitivity and the like as previously described. For example, in a situation in which a non-ionic surfactant including a polyoxyethylene chain (polyoxyethylene sorbitan fatty acid ester, polyoxyethylene sorbitol fatty acid ester, polyoxyethylene glycerin fatty acid ester, polyoxyethylene fatty acid ester, polyoxyethylene alkyl ether, polyoxyethylene polyoxypropylene alkyl ether, polyoxyethylene alkyl phenyl ether, polyoxyethylene hydrogenated castor oil, polyoxyethylene beeswax derivative, polyoxyethylene lanolin derivative, polyoxyethylene fatty acid amide, etc.) is stored for a long time, the end of the polyoxyethylene chain may be severed through self-oxidation to thereby produce a decomposition product. However, since the non-ionic surfactant concentration in the protein solution formulation is not less than 0 mg/mL and less than 0.05 mg/mL in the present disclosure as previously described, even if a decomposition product were to be produced, the produced amount thereof would be very small, and thus the risk of hypersensitivity, chromosome abnormalities, carcinogenesis, and the like described above can be sufficiently reduced.

From a viewpoint of reduction of the risks described above, the non-ionic surfactant concentration in the protein solution formulation is preferably 0.04 mg/mL or less, more preferably 0.01 mg/mL or less, even more preferably 0.005 mg/mL or less, and particularly preferably 0 mg/mL (below the limit of detection).

<<Other components>>

The protein solution formulation may contain components other than a protein, water, and a non-ionic surfactant (i.e., other components). Examples of other components that are optionally contained in the protein solution formulation include known components that can be used in production of protein solution formulations. Examples of such known components include stabilizers (excluding the non-ionic surfactants described above), diluents, solubilizers, tonicity agents, excipients, pH modifiers, numbing agents, buffering agents, sulfur-containing reducing agents, and antioxidants. Further examples of other components include inorganic salts such as sodium chloride, potassium chloride, calcium chloride, sodium phosphate, potassium phosphate, and sodium hydrogen carbonate; and organic salts such as sodium citrate, potassium citrate, and sodium acetate. Note that the inorganic salt concentration in the protein solution formulation is preferably 300 mM or less. Moreover, the organic salt concentration in the protein solution formulation is preferably 300 mM or less.

<<Production method>>

No specific limitations are placed on the method by which the protein solution formulation is produced so long as it is possible to obtain a protein solution formulation in which at least a protein is dissolved and in which the non-ionic surfactant concentration is within a specific range. For example, the protein solution formulation can be obtained by dissolving the protein and a surfactant that is used as necessary in an aqueous buffer solution such as an acetate buffer solution, a phosphate buffer solution, or a citrate buffer solution.
The pH of the obtained protein solution formulation is not specifically limited and can be set as not lower than 3.0 and not higher than 8.0.

<Housing>

The housing included in the presently disclosed pre-filled drug package is not specifically limited so long as it is a member having a space (internal space) that can accommodate the protein solution formulation in an inner part thereof and may be a member having an internal space that is defined by only the housing itself. However, as previously described, it is preferable that the housing has an opening that connects the internal space with the outside and that this opening is hermetically sealed by a sealing member as illustrated in FIG. 1 and FIG. 2, described above, from viewpoints such as facilitating removal of the protein solution formulation.
At least part of a formulation contacting section of the housing is required to be formed of a resin containing either or both of a hydrogenated cycloolefin ring-opened polymer and a copolymer of a cycloolefin and a chain olefin as previously described.
In other words, it is necessary for a shaped product of the specific resin described above (resin shaped product) to be positioned at at least an inner wall surface of the housing that forms the internal space and for the resin shaped product and the protein solution formulation to be directly in contact without another member (for example, a coating film not formed of the specific resin shaped product) in-between.
In order that the resin shaped product and the protein solution formulation are in contact in this manner, the resin shaped product may constitute the entirety of the housing or may constitute part of the housing. For example, a wall that forms the internal space of the housing may have a structure (multilayer structure) in which a plurality of layers are stacked and in which at least an innermost layer is the resin shaped product.
From a viewpoint of further inhibiting protein aggregation in the protein solution formulation during long-term storage of the pre-filled drug package, it is preferable that the entirety of the formulation contacting section of the housing is formed of a resin containing either or both of a hydrogenated cycloolefin ring-opened polymer and a copolymer of a cycloolefin and a chain olefin.
The resin used to form the housing is described below. Note that the resin used to form the housing may contain components other than the hydrogenated cycloolefin ring-opened polymer and the copolymer of a cycloolefin and a chain olefin mentioned above (i.e., other components).

<<Hydrogenated cycloolefin ring-opened polymer>>

The hydrogenated cycloolefin ring-opened polymer is a polymer that is obtained by performing ring-opening polymerization of a cycloolefin as a monomer to obtain a cycloolefin ring-opened polymer, and then further subjecting the obtained cycloolefin ring-opened polymer to a hydrogenation reaction.

[Cycloolefin ring-opened polymer]

A compound that has a cyclic structure formed of carbon atoms and includes a polymerizable carbon-carbon double bond in the cyclic structure can be used as a cycloolefin serving as a monomer in production of the cycloolefin ring-opened polymer. Specifically, the cycloolefin serving as a monomer may be a norbornene-based monomer (monomer including a norbornene ring) or a monocyclic cycloolefin monomer. Note that in a "norbornene ring" included in a norbornene-based monomer, one or a plurality of carbon atoms may be interposed between carbon-carbon single bonds that form the ring structure, and these interposed carbon atoms may form single bonds with one another, resulting in the formation of a new ring structure in the norbornene ring.

Examples of norbornene-based monomers include:

bicyclic monomers such as bicyclo[2.2.1]hept-2-ene (common name: norbornene) and derivatives thereof (derivatives including a substituent on a ring; same applies below), and 5-ethylidene-bicyclo[2.2.1]hept-2-ene (common name: ethylidene norbornene) and derivatives thereof;

tricyclic monomers such as tricyclo[4.3.0.1$^{2,5}$]deca-3,7-diene (common name: dicyclopentadiene) and derivatives thereof; and

tetracyclic monomers such as 7,8-benzotricyclo[4.3.0.1$^{2,5}$]dec-3-ene (common name: methanotetrahydrofluorene and derivatives thereof; also referred to as tetracyclo[7.4.0.0$^{2,7}$.1$^{10,13}$]trideca-2,4,6,11-tetraene) and derivatives thereof, tetracyclo[4.4.0.1$^{2,5}$.1$^{7,10}$]dodec-3-ene (common name: tetracyclododecene) and derivatives thereof (for example, 8-methyl-tetracyclo[4.4.0.1$^{2,5}$.1$^{7,10}$]dodec-3-ene and 8-ethyl-tetracyclo[4.4.0.1$^{2,5}$.1$^{7,10}$]dodec-3-ene), and 8-ethylidenetetracyclo[4.4.0.1$^{2,5}$.1$^{7,10}$]-3-dodecene and derivatives thereof.

Examples of possible substituents of the aforementioned derivatives include alkyl groups such as a methyl group and an ethyl group; alkenyl groups such as a vinyl group; alkylidene groups such as an ethylidene group and a propan-2-ylidene group; aryl groups such as a phenyl group; a hydroxy group; an acid anhydride group; a carboxyl group; and alkoxycarbonyl groups such as a methoxycarbonyl group.

Examples of monocyclic cycloolefin monomers include cyclic monoolefins such as cyclobutene, cyclopentene, methylcyclopentene, cyclohexene, methylcyclohexene, cycloheptene, and cyclooctene; and cyclic diolefins such as cyclohexadiene, methylcyclohexadiene, cyclooctadiene, methylcyclooctadiene, and phenylcyclooctadiene.

One of the cycloolefins described above may be used individually, or two or more of the cycloolefins described above may be used in combination. Note that in a case in which two or more cycloolefins are used, the cycloolefin ring-opened polymer may be a block copolymer or may be a random copolymer.

Of these examples, norbornene-based monomers are preferable, tricyclo[4.3.0.1$^{2,5}$]deca-3,7-diene and derivatives thereof, tetracyclo[4.4.0.1$^{2,5}$.1$^{7,10}$]dodec-3-ene and derivatives thereof, and 7,8-benzotricyclo[4.3.0.1$^{2,5}$]dec-3-ene and derivatives thereof are more preferable, and tricyclo[4.3.0.1$^{2,5}$]deca-3,7-diene, tetracyclo[4.4.0.1$^{2,5}$.1$^{7,10}$]dodec-3-ene, and 7,8-benzotricyclo[4.3.0.1$^{2,5}$]dec-3-ene are even more preferable as the cycloolefin.

Although no specific limitations are placed on the amount of a norbornene-based monomer that is used in production of the cycloolefin ring-opened polymer, the amount of the norbornene-based monomer per 100 mass% of the amount of all cycloolefin used in production of the cycloolefin ring-opened polymer is preferably 80 mass% or more, more preferably 90 mass% or more, and even more preferably 100 mass% (i.e., the cycloolefin ring-opened polymer is even more preferably a polymer obtained using only one or more norbornene-based monomers as monomers).

No specific limitations are placed on the method by which the cycloolefin ring-opened polymer is produced. For example, a known method in which a cycloolefin such as described above that is used as a monomer is ring-opening polymerized using a metathesis polymerization catalyst can be adopted. This method may, for example, be a method described in JP2016-155327A.

The weight-average molecular weight (Mw) of the cycloolefin ring-opened polymer obtained as set forth above is not specifically limited but is preferably 10,000 or more, and more preferably 15,000 or more, and is preferably 100,000 or less, and more preferably 50,000 or less. When the weight-average molecular weight of the cycloolefin ring-opened polymer is 10,000 or more, it is possible to ensure sufficient strength of a housing obtained using a resin that contains a hydrogenated product of the cycloolefin ring-opened polymer. On the other hand, when the weight-average molecular weight of the cycloolefin ring-opened polymer is 100,000 or less, it is possible to ensure sufficient formability of a resin that contains a hydrogenated product of the cycloolefin ring-opened polymer.

Moreover, the molecular weight distribution (Mw/Mn) of the cycloolefin ring-opened polymer is not specifically limited but is preferably not less than 1 and not more than 5, and more preferably not less than 1 and not more than 4. When the molecular weight distribution of the cycloolefin ring-opened polymer is within any of the ranges set forth above, a housing having sufficient mechanical strength can be obtained.

Note that the weight-average molecular weight (Mw) and number-average molecular weight (Mn) of a polymer such as a cycloolefin ring-opened polymer referred to in the present disclosure are standard polyisopreneequivalent values according to gel permeation chromatography (GPC) with cyclohexane as an eluent.

[Hydrogenation reaction]

The hydrogenated cycloolefin ring-opened polymer can be obtained by subjecting the cycloolefin ring-opened polymer described above to a hydrogenation reaction. No specific limitations are placed on the method by which the cycloolefin ring-opened polymer is hydrogenated. For example, a known method in which hydrogen is supplied into a reaction system in the presence of a hydrogenation catalyst can be adopted. This method may, for example, be a method described in JP2016-155327A.

The percentage hydrogenation in the hydrogenation reaction (proportion of main chain carbon-carbon double bonds that are hydrogenated) is not specifically limited but is preferably 70% or more, more preferably 80% or more, even more preferably 90% or more, and particularly preferably 99% or more from a viewpoint of inhibiting the occurrence of burns and oxidative degradation during production of a housing through shaping of the hydrogenated cycloolefin ring-opened polymer.

Note that the "percentage hydrogenation" in a hydrogenation reaction referred to in the present disclosure can be measured by nuclear magnetic resonance (NMR).

The weight-average molecular weight (Mw) of the hydrogenated cycloolefin ring-opened polymer obtained after the hydrogenation reaction described above is not specifically limited but is preferably 10,000 or more, and more preferably 15,000 or more, and is preferably 100,000 or less, and more preferably 50,000 or less. When the weight-average molecular weight of the hydrogenated cycloolefin ring-opened polymer is 10,000 or more, it is possible to ensure sufficient strength of a housing obtained using a resin that contains the hydrogenated cycloolefin ring-opened polymer. On the other hand, when the weight-average molecular weight of the hydrogenated cycloolefin ring-opened polymer is 100,000 or less, it is possible to ensure sufficient formability of a resin that contains the hydrogenated cycloolefin ring-opened polymer.

Moreover, the molecular weight distribution (Mw/Mn) of the hydrogenated cycloolefin ring-opened polymer is not specifically limited but is preferably not less than 1 and not more than 5, and more preferably not less than 1 and not more than 4. When the molecular weight distribution of the hydrogenated cycloolefin ring-opened polymer is within any of the ranges set forth above, a housing having sufficient mechanical strength can be obtained.

<<Copolymer of cycloolefin and chain olefin>>

The copolymer of a cycloolefin and a chain olefin (hereinafter, also referred to simply as the "copolymer") is a polymer that is obtained through copolymerization of a cycloolefin as a monomer and a chain olefin as a monomer.

[Cycloolefin]

Any of the same cycloolefins as previously described in the "Hydrogenated cycloolefin ring-opened polymer" section can be used as the cycloolefin serving as a monomer used in production of the copolymer. One cycloolefin may be used individually, or two or more cycloolefins may be used in combination. Of these cycloolefins, bicyclo[2.2.1]hept-2-ene (common name: norbornene) and derivatives thereof, and tetracyclo[4.4.0.1$^{2,5}$.1$^{7,10}$]dodec-3-ene (common name: tetracyclododecene) and derivatives thereof are preferable, and bicyclo[2.2.1]hept-2-ene is more preferable.

[Chain olefin]

A compound that has a chain structure formed of carbon atoms and includes a polymerizable carbon-carbon double bond in the chain structure can be used as the chain olefin serving as a monomer in production of the copolymer. Note that compounds corresponding to the cycloolefin are not considered to be included among examples of the chain olefin.

The chain olefin may, for example, be an $\alpha$-olefin such as ethylene, propylene, 1-butene, 1-pentene, or 1-hexene; an aromatic ring vinyl compound such as styrene or $\alpha$-methylstyrene; or a non-conjugated diene such as 1,4-hexadiene, 4-methyl-1,4-hexadiene, 5-methyl-1,4-hexadiene, or 1,7-octadiene.

One chain olefin may be used individually, or two or more chain olefins may be used in combination. Of these examples, $\alpha$-olefins are preferable, $\alpha$-olefins having a carbon number of not less than 1 and not more than 20 are more preferable, and ethylene is even more preferable as the chain olefin.

[Copolymer]

No specific limitations are placed on the method by which the copolymer is produced. For example, a known method in which the cycloolefin and the chain olefin described above are addition polymerized using a polymerization catalyst can be adopted. This method may, for example, be a method described in JP2016-155327A.

Although no specific limitations are placed on the ratio of amounts of the cycloolefin and the chain olefin used in production of the copolymer, the amount of the cycloolefin per 100 mass% of the total amount of the cycloolefin and the chain olefin used in production of the copolymer is preferably 30 mass% or more, more preferably 50 mass% or more, and even more preferably 70 mass% or more, and is preferably 99 mass% or less, more preferably 97 mass% or less, and even more preferably 95 mass% or less.

Note that the copolymer of the cycloolefin and the chain olefin may be a block copolymer or may be a random copolymer.

The weight-average molecular weight (Mw) of the copolymer of the cycloolefin and the chain olefin is not specifically limited but is preferably 20,000 or more, and more preferably 25,000 or more, and is preferably 100,000 or less. When the weight-average molecular weight of the copolymer is 20,000 or more, it is possible to ensure sufficient strength of a housing obtained using a resin that contains the copolymer. On the other hand, when the weight-average molecular weight of the copolymer is 100,000 or less, it is possible to ensure sufficient formability of a resin that contains the copolymer.

Moreover, the molecular weight distribution (Mw/Mn) of the copolymer is not specifically limited but is preferably not less than 1 and not more than 5, and more preferably not less than 1 and not more than 4. When the molecular weight distribution of the copolymer is within any of the ranges set forth above, a housing having sufficient mechanical strength can be obtained.

<<Preferred polymer>>

The resin used to form the housing contains either or both of a hydrogenated cycloolefin ring-opened polymer and a copolymer of a cycloolefin and a chain olefin as previously described, but preferably contains at least a hydrogenated cycloolefin ring-opened polymer. By using a housing that is obtained using a resin containing at least a hydrogenated cycloolefin ring-opened polymer, it is possible to sufficiently inhibit protein aggregation in the protein solution formulation during long-term storage of the pre-filled drug package.

<<Other components>>

Examples of other components that can optionally be contained in the resin used to form the housing include polymer components (thermoplastic elastomers, etc.) other than the polymers described above and known additives. Examples of known additives that can be used include antioxidants, ultraviolet absorbers, light stabilizers, near-infrared absorbers, plasticizers, antistatic agents, acid scavengers, and the like described in JP2016-155327A, for example. Just one of these other components may be used, or a combination of two or more of these other components may be used.

The content of these other components in the resin can be set as appropriate depending on the objective of addition of the component. For example, in a case in which a thermoplastic elastomer is used, the content of the thermoplastic elastomer is preferably not less than 0.01 parts by mass and not more than 0.5 parts by mass when the total amount of the hydrogenated cycloolefin ring-opened polymer and the copolymer of a cycloolefin and a chain olefin is taken to be 100 parts by mass (i.e., when the amount of either of these polymers is taken to be 100 parts by mass in a case in which that polymer is used individually).

Moreover, it is preferable that either or both of an antioxidant and a light stabilizer are used as additives from a viewpoint of further inhibiting protein aggregation in the protein solution formulation.

The antioxidant is, for example, preferably a phenolic antioxidant such as pentaerythritol tetrakis[3-(3,5-di-t-butyl-4-hydroxyphenyl)propionate]; or a phosphoric antioxidant such as 6-[3-(3-t-butyl-4-hydroxy-5-methylphenyl)propoxy]-2,4,8,10-tetrakis-t-butyldibenzo[d,f][1.3.2]dioxaphosphepine or 3,9-bis(2,6-di-tert-butyl-4-methylphenoxy)-2,4,8,10-tetraoxa-3,9-diphosphaspiro[5.5]undecane.

The light stabilizer is, for example, preferably a hindered amine light stabilizer such as dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine polycondensate.

Each of the antioxidant and the light stabilizer may be just one type or may be a combination of two or more types. Moreover, an antioxidant and a light stabilizer can be used in combination.

The content of the antioxidant in the resin is preferably not less than 0.01 parts by mass and not more than 0.2 parts by mass when the total amount of the hydrogenated cycloolefin ring-opened polymer and the copolymer of a cycloolefin and a chain olefin is taken to be 100 parts by mass (i.e., when the amount of either of these polymers is taken to be 100 parts by mass in a case in which that polymer is used individually) from a viewpoint of further inhibiting protein aggregation in the protein solution formulation.

Moreover, the content of the light stabilizer in the resin is preferably not less than 0.02 parts by mass and not more than 0.5 parts by mass when the total amount of the hydrogenated cycloolefin ring-opened polymer and the copolymer of a cycloolefin and a chain olefin is taken to be 100 parts by mass (i.e., when the amount of either of these polymers is taken to be 100 parts by mass in a case in which that polymer is used individually) from a viewpoint of further inhibiting protein aggregation in the protein solution formulation.

No specific limitations are placed on the method of mixing adopted when obtaining the resin containing the above-described polymer(s) and other optional components. For example, mixing can be performed using a known melt-kneading machine such as a single-screw extruder, a twin-screw extruder, a Banbury mixer, a kneader, or a Feeder Ruder.

After mixing, the resin can be pelletized in accordance with a standard method by extruding the resin with a rod form and cutting the extruded resin to an appropriate length using a strand cutter.

«Production method of housing»

No specific limitations are placed on the method by which the resin containing the components set forth above is shaped to obtain the housing having a resin shaped product constituting at least part of an inner wall surface thereof. For example, a method described in the "Method of producing pre-filled drug package" section further below can be adopted.

<<Water contact angle>>

In the housing obtained as set forth above, the water contact angle of a formulation contacting section is preferably 90° or more, and more preferably 91° or more from a viewpoint of further inhibiting protein aggregation in the protein solution formulation. Note that the water contact angle of the formulation contacting section can be set as 110° or less, can be set as 100° or less, or can be set as 94° or less, for example, without any specific limitations.

Also note that the water contact angle of the formulation contacting section can be adjusted by altering the types of polymers and additives contained in the resin used to form the housing and the method by which the housing is produced. For example, the value of the water contact angle of the formulation contacting section can be improved by using a hydrophobic polymer or additive (polymer or additive that does not include a hydrophilic group, for example). Moreover, the value of the water contact angle of the formulation contacting section can be improved by performing pre-drying described in the "Method of producing pre-filled drug package" section further below in advance of shaping the resin, for example.

<Sealing member>

The presently disclosed pre-filled drug package optionally includes a sealing member. The sealing member is not specifically limited so long as it is a member that, in a case in which the housing set forth above has an opening, can hermetically seal the opening and prevent leakage of the protein solution formulation accommodated in the internal space, and may be a known sealing member such as a cap or a port.

The material of the sealing member is not specifically limited and can be a known resin material such as a rubber, for example. Moreover, a resin containing either or both of a hydrogenated cycloolefin ring-opened polymer and a copolymer of a cycloolefin and a chain olefin may be used as the material of the sealing member from a viewpoint of further inhibiting protein aggregation in the protein solution formulation.

(Method of producing pre-filled drug package)

The presently disclosed pre-filled drug package set forth above can more suitably be produced by the presently disclosed method of producing a pre-filled drug package, for example.

The presently disclosed method of producing a pre-filled drug package is a method of producing a pre-filled drug package that includes a protein solution formulation and a housing accommodating the protein solution formulation. The presently disclosed method of producing a pre-filled drug package includes at least a step (filling step) of filling a protein solution formulation having a non-ionic surfactant concentration of not less than 0 mg/mL and less than 0.05 mg/mL into a housing to obtain a pre-filled drug package accommodating the protein solution formulation. At least part of a formulation contacting section of the housing is formed using a resin containing either or both of a hydrogenated cycloolefin ring-opened polymer and a copolymer of a cycloolefin and a chain olefin.

The pre-filled drug package that is obtained by filling with the protein solution formulation having a non-ionic surfactant concentration of not less than 0 mg/mL and less than 0.05 mg/mL through the filling step set forth above can inhibit protein aggregation in the protein solution formulation even upon long-term storage for the same reason as previously described in the "Pre-filled drug package" section.

Note that the "housing", "hydrogenated cycloolefin ring-opened polymer", "copolymer of a cycloolefin and a chain olefin", "resin", "sealing member", "protein solution formulation", and so forth in the following description are the same as previously described in the "Pre-filled drug package" section. In other words, specific examples, preferable examples, and so forth of the "housing", "hydrogenated cycloolefin ring-opened polymer", "copolymer of a cycloolefin and a chain olefin", "resin", "sealing member", and "protein solution formulation" in the presently disclosed method of producing a pre-filled drug package are the same as the specific examples, preferable examples, and so forth of the "housing", "hydrogenated cycloolefin ring-opened polymer", "copolymer of a cycloolefin and a chain olefin", "resin", "sealing member", and "protein solution formulation" in the presently disclosed pre-filled drug package set forth above, and thus description thereof is omitted in this section.

<Filling step>

The method by which the protein solution formulation is filled into the internal space of the housing is not specifically limited and can be a known method. For example, in a case in which the housing has an opening, the protein solution formulation may be loaded through the opening, and then the opening may be sealed by the sealing member. The filling step is preferably performed under sterilization.

<Other steps>

The presently disclosed method of producing a pre-filled drug package can optionally include steps other than the filling step set forth above (i.e., other steps).

The presently disclosed method of producing a pre-filled drug package preferably includes, in advance of the filling step set forth above, a step (pre-drying step) of pre-drying the resin that serves as a shaping material, and a step (shaping step) of shaping the resin after the pre-drying to shape the housing.

<<Pre-drying step>>

By drying the resin that is used to form the housing in advance of shaping the resin, the water contact angle of the surface of the housing (particularly the surface of a formulation contacting section) can be maintained, and protein aggregation in the protein solution formulation can be sufficiently inhibited. Note that although it is not clear why the water contact angle of the surface of the housing that is obtained after shaping can be maintained by drying the resin before shaping, it is presumed that the drying can reduce the oxygen concentration in the resin, and can thereby inhibit oxidation and hydrophilizing of the housing surface due to heat during shaping.

Note that no specific limitations are placed on the shape of the resin during the pre-drying. The resin can have any shape such as a sheet shape or a pellet shape, but preferably has a pellet shape from a viewpoint of drying efficiency and ease of shaping.

The oxygen concentration in the resin after the pre-drying is preferably 10 mass ppm or less, more preferably 5 mass ppm or less, even more preferably 4 mass ppm or less, and particularly preferably 1.5 mass ppm or less. When the oxygen concentration in the resin after the pre-drying is 10 mass ppm or less, the value of the water contact angle of the formulation contacting section of the housing formed using the resin can be maintained, and thus protein aggregation in the protein solution formulation can be further inhibited in a pre-filled drug package that includes the housing.

The pre-drying is preferably performed in an inert gas atmosphere. By performing the pre-drying in an inert gas atmosphere, oxygen can be efficiently removed from the resin while also preventing oxidation of the resin due to external oxygen. As a result, protein aggregation in the protein solution formulation can be further inhibited in a pre-filled drug package that includes the obtained housing. Examples of inert gases that can be used include helium, argon, nitrogen, neon, krypton, and mixtures thereof.

The drying temperature (atmosphere temperature) of the pre-drying is preferably 80°C or higher, more preferably 90°C or higher, and even more preferably 100°C or higher, and is preferably 120°C or lower, and more preferably 110°C or lower. When the drying temperature of the pre-drying is 80°C or higher, oxygen can be efficiently removed from the resin, and, as a result, protein aggregation in the protein solution formulation can be further inhibited in a pre-filled drug package that includes the obtained housing. On the other hand, when the drying temperature of the pre-drying is 120°C or lower, curing of the resin prior to shaping can be prevented.

The drying time of the pre-drying is preferably 1 hour or more, more preferably 2 hours or more, and even more preferably 4 hours or more, and is preferably 24 hours or less, and more preferably 12 hours or less. When the drying time of the pre-drying is 1 hour or more, oxygen can be efficiently removed from the resin, and, as a result, protein aggregation in the protein solution formulation can be further inhibited in a pre-filled drug package that includes the obtained housing. On the other hand, when the drying time of the pre-drying is 24 hours or less, oxidative degradation of the resin prior to shaping can be prevented.

<<Shaping step>>

The method by which the resin after the pre-drying set forth above is shaped is not specifically limited and can be selected as appropriate from known shaping methods depending on the type of pre-filled drug package and the desired shape of the housing. Examples of such known shaping methods include extrusion molding, injection molding, inflation molding, blow molding, extrusion blow molding, injection blow molding, press forming, vacuum forming, powder slush molding, calendering, foam molding, and thermoforming. Shaping conditions in these known shaping methods can be set as appropriate.

In a case in which a wall forming the internal space of the housing has a multilayer structure, the housing including the internal space can be obtained by, for example, producing a multilayer film having that multilayer structure, and then adhering the perimeters of a pair of such multilayer films.

Note that the presently disclosed method of producing a pre-filled drug package can include a step of sterilizing the housing and/or the sealing member before the filling step, for example, as another step besides the pre-drying step and the shaping step set forth above.

EXAMPLES

The following provides a more specific description of the present disclosure through examples. However, the present disclosure is not limited to these examples. In the following description, "%" and "parts" used in expressing quantities are by mass, unless otherwise specified.

In the examples and comparative examples, the following methods were used to measure and evaluate the molecular weight, etc. (weight-average molecular weight, number-average molecular weight, and molecular weight distribution) of a polymer, the percentage hydrogenation when a polymer is hydrogenated, the glass-transition temperature of a polymer, the oxygen concentration in a resin, the water contact angle of a formulation contacting section of a housing, the non-ionic surfactant concentration in a protein solution formulation after long-term storage of a pre-filled drug package (post-storage concentration), the occurrence of production of a decomposition product of a non-ionic surfactant in a protein solution formulation after long-term storage of a pre-filled drug package, and the inhibition of protein aggregation in a protein solution formulation after long-term storage of a pre-filled drug package.

<Molecular weight, etc.>

The weight-average molecular weight (Mw) and the number-average molecular weight (Mn) of a polymer were measured as standard polyisopreneequivalent values by gel permeation chromatography (GPC) with cyclohexane as a solvent. The molecular weight distribution (Mw/Mn) was calculated from the obtained values of Mw and Mn. An HLC-8320GPC (produced by Tosoh Corporation) was used as a measurement apparatus. The standard polyisoprene was standard polyisoprene (monodisperse) produced by Tosoh Corporation, and a total of 10 points corresponding to weight-average molecular weights (Mw) of 602, 1,390, 3,920, 8,050, 13,800, 22,700, 58,800, 71,300, 109,000, and 280,000 were used. Measurement was performed with a TSKgel G5000HXL, a TSKgel G4000HXL, and a TSKgel G2000HXL (each produced by Tosoh Corporation) connected in series as a column and under conditions of a flow rate of 1.0 mL/min, a sample injection volume of 100 μL, and a column temperature of 40°C.

<Percentage hydrogenation>

The percentage hydrogenation in a hydrogenation reaction was calculated through $^1$H-NMR measurement with deuterated chloroform as a solvent.

<Glass-transition temperature (Tg)>

Glass-transition temperature measurement was performed based on JIS K 6911 using a differential scanning calorimeter (produced by SII Nanotechnology; product name: DSC6220).

<Oxygen concentration in resin>

A thermal desorption analyzer (produced by ESCO, Ltd.; product name: WA1000S/W) was used to heat resin pellets at 130°C for 60 minutes and to measure the amount of desorbed oxygen during this heating in order to calculate the oxygen concentration in the resin.

<Water contact angle>

A housing or multilayer film was cut by diagonal pliers to cut out a formulation contacting section thereof. A contact angle meter (produced by Kyowa Interface Science Co., Ltd.; product name: Drop Master 300) was used to measure the static contact angle by a curve fitting method for 10 arbitrarily selected locations in the formulation contacting section, and an average value of the measured values was taken to be the water contact angle of the formulation contacting section.

<Post-storage concentration of non-ionic surfactant>

A pre-filled drug package was left at rest in the dark at 4°C for 1 week. After the pre-filled drug package had been left at rest for 1 week, the protein solution formulation in the pre-filled drug package was collected.
The non-ionic surfactant concentration in the protein solution formulation after long-term storage (post-storage concentration) was calculated by the following formula.

$$\text{Post-storage concentration (mg/mL)} = \text{Non-ionic surfactant concentration before long-term storage (mg/mL; initial concentration)} \times (A1/A0)$$

Note that A0 and A1 in the preceding formula are the area intensities of peaks attributed to a non-ionic surfactant that were obtained from data acquired through high-performance liquid chromatography (HPLC) analysis of the protein solution formulation before long-term storage and after long-term storage, respectively. The conditions under which HPLC was performed were as follows.

Apparatus: HP-1100 (product name; produced by Agilent Technologies, Inc.)
Column: ZORBAX® (ZORBAX is a registered trademark in Japan, other countries, or both) Eclipse Plus C18HT (product name; 2.1 mm i.d. × 150 mm, 1.8 μm; produced by Agilent Technologies, Inc.)
Solvent: Acetonitrile/water = 70/30
Injection volume: 16 μL
Flow rate: 0.4 mL/min
Detection method: UV 198 nm

<Occurrence of production of decomposition product of non-ionic surfactant>

A pre-filled drug package was left at rest in the dark at 4°C for 1 week. After the pre-filled drug package had been left at rest for 1 week, the protein solution formulation in the pre-filled drug package was collected.
HPLC analysis of the protein solution formulation after long-term storage was performed under the same conditions as in "Post-storage concentration of non-ionic surfactant", and the presence or absence of a peak attributed to a decomposition product was checked. Note that a judgment of "Yes" for production of a decomposition product after storage was made in a case in which such a peak was observed, whereas a judgment of "No" for production of a decomposition product after storage (i.e., that the produced amount was below the limit of detection) was made in a case in which such a peak was not observed.

<Inhibition of protein aggregation>

A pre-filled drug package was left at rest in the dark at 4°C for 1 week. After the pre-filled drug package had been left at rest for 1 week, the protein solution formulation in the pre-filled drug package was collected. The number of aggregates having a particle diameter of 1 μm or more that were contained in the collected protein solution formulation was visually counted using a FlowCam 8100 (Fluid Imaging Technologies, Scarborough, ME). Note that the sample volume was 0.15 mL, and analysis was performed at a flow rate of 0.05 mL/min. Data analysis was performed using Visual Spreadsheet Software (Fluid Imaging Technologies). The same operations were performed a total of four times. The number of aggregates per unit volume (aggregates/mL) was calculated for each repetition of the operations, and an average value of the calculated values was taken to be the post-storage aggregate concentration (aggregates/mL). A smaller value for the post-storage aggregate concentration can be said to signify that protein aggregation in the protein solution formulation during long-term storage of the pre-filled drug package is inhibited.

(Example 1-1)

<Preparation of protein solution formulation>

Purified Humira (adalimumab) was adjusted to a concentration of 0.1 mg/mL to obtain a protein solution formulation using phosphate buffered saline (pH: 7.0; NaCl: 200 mM; phosphoric acid: 100 mM).

<Production method of housing>

<<Production of hydrogenated cycloolefin ring-opened polymer (hydrogenated product A)>>

In a nitrogen atmosphere, 0.82 parts of 1-hexene, 0.15 parts of dibutyl ether, and 0.30 parts of triisobutylaluminum were added to 500 parts of dehydrated cyclohexane in a reactor at room temperature and were mixed therewith. Thereafter, the mixture was held at 45°C while 76 parts of tricyclo[4.3.0.1$^{2,5}$]deca-3,7-diene (common name: dicyclopentadiene; hereinafter, abbreviated as "DCP"), 70 parts of tetracyclo[4.4.0.1$^{2,5}$.1$^{7,10}$]dodec-3-ene (hereinafter, abbreviated as "TCD"), 54 parts of tetracyclo[7.4.0.0$^{2,7}$.1$^{10,13}$]trideca-2,4,6,11-tetraene (hereinafter, abbreviated as "MTF"), and 80 parts of tungsten hexachloride (0.7% toluene solution) were continuously added over 2 hours, concurrently to one another, and polymerization was carried out. Next, 1.06 parts of butyl glycidyl ether and 0.52 parts of isopropyl alcohol were added to the polymerization solution to deactivate the polymerization catalyst and stop the polymerization reaction. When the resultant reaction solution containing a ring-opened polymer was analyzed by gas chromatography, the polymerization conversion rate of monomers was 99.5%.
Next, 270 parts of cyclohexane was added to 100 parts of the obtained reaction solution containing the ring-opened polymer, 5 parts of diatomitesupported nickel catalyst (nickel support rate: 58 weight%; pore volume: 0.25 mL/g; specific surface area: 180 m$^2$/g) was further added as a hydrogenation catalyst, the pressure was raised to 5 MPa with hydrogen, heating was performed to a temperature of 200°C under stirring, and then a reaction was carried out for 8 hours to obtain a reaction solution containing a hydrogenated DCP/TCD/MTF ring-opened copolymer. The hydrogenation catalyst was removed by filtration, and then cyclohexane serving as a solvent and other volatile components were removed from the solution at a temperature of 270°C and a pressure of 1 kPa or lower using a cylindrical evaporator (produced by Hitachi, Ltd.). Next, the hydrogenated product was extruded in a strand form from an extruder while in a molten state, was cooled, and was subsequently pelletized to obtain pellets. The hydrogenated cycloolefin ring-opened polymer (hydrogenated product A) that had been pelletized had an Mw of 34,000, a molecular weight distribution (Mw/Mn) of 2.3, a percentage hydrogenation of 99.7%, and a Tg of 137°C.

<<Production of resin pellets containing hydrogenated product A>>

After mixing 100 parts of the hydrogenated product A obtained as described above and 0.024 parts of pentaerythritol tetrakis[3-(3,5-di-t-butyl-4-hydroxyphenyl)propionate] (hereinafter, abbreviated as "antioxidant X") as an antioxidant using a blender, a twin-screw kneader for which hopper purging with nitrogen had been performed was used to knead and extrude the mixture at a cylinder temperature of 285°C to obtain resin pellets.

<<Pre-drying and shaping>>

A hot-air dryer was used to dry (pre-dry) the resin pellets obtained as described above in a nitrogen atmosphere under conditions of an atmosphere temperature of 100°C and a drying time of 6 hours. The oxygen concentration of the resin after this pre-drying was measured. The result is shown in Table 1.
The pre-dried resin was injection molded with a resin temperature of 290°C and a mold temperature of 100°C using an injection molding machine (produced by FANUC Corporation; product name: FANUC ROBOSHOT® α100B (FANUC ROBOSHOT is a registered trademark in Japan, other countries, or both)) so as to produce a preform having a total length of 34.5 mm, a maximum outer diameter of 11.0 mm, and a wall thickness of 3.0 mm. This preform was stretched lengthwise by a factor of 1.1 and widthwise by a factor of 2.0 using a blow molding machine (produced by Frontier Inc.; product name: FMB-1) under conditions of a preform heating temperature of 185°C, a blow time of 1.10 seconds, and a mold temperature of 137°C so as to produce a housing of a roughly circular tube shape having an opening. The water contact angle of a formulation contacting section was measured for the obtained housing. The result is shown in Table 1.

<Production of pre-filled drug package (vial)>

The housing and the protein solution formulation described above were used to produce a pre-filled drug package having the configuration illustrated in FIG. 1 by the following procedure. Note that production of the pre-filled drug package was performed in a sterilized environment.

The internal space of the housing was filled with 10.0 mL of the protein solution formulation through the opening of the housing. Next, the opening of the housing was hermetically sealed by a rubber stopper (obtained through lamination of PTFE film) as a sealing member to obtain a pre-filled drug package that had been filled with the protein solution formulation. The obtained pre-filled drug package was used to evaluate the post-storage concentration of a non-ionic surfactant, the occurrence of production of a decomposition product of a non-ionic surfactant, and the inhibition of protein aggregation. The results are shown in Table 1.

(Example 1-2)

A pre-filled drug package was produced and various evaluations were performed in the same way as in Example 1-1 with the exception that 0.04 parts of 6-[3-(3-t-butyl-4-hydroxy-5-methylphenyl)propoxy]-2,4,8,10-tetrakis-t-butyldibenzo[d,f][1.3.2]dioxaphosphepine (hereinafter, abbreviated as "antioxidant Y") was instead of the antioxidant X in production of the housing. The results are shown in Table 1.

(Example 1-3)

A pre-filled drug package was produced and various evaluations were performed in the same way as in Example 1-1 with the exception that 0.15 parts of dibutylamine, 2,4,6-trichloro-1,3,5-triazine, N,N'-bis(2,2,6,6-tetramethyl-4-piperidyl)-1,6-hexamethylenediamine, and N-(2,2,6,6-tetramethyl-4-piperidyl)butylamine polycondensate (hereinafter, abbreviated as "light stabilizer Z") was used instead of the antioxidant X in production of the housing. The results are shown in Table 1.

(Example 1-4)

A pre-filled drug package was produced and various evaluations were performed in the same way as in Example 1-1 with the exception that 0.04 parts of the antioxidant Y and 0.15 parts of the light stabilizer Z were used instead of the antioxidant X in production of the housing. The results are shown in Table 1.

(Example 1-5)

A pre-filled drug package was produced and various evaluations were performed in the same way as in Example 1-1 with the exception that 0.2 parts of 3,9-bis(2,6-di-tert-butyl-4-methylphenoxy)-2,4,8,10-tetraoxa-3,9-diphosphaspiro[5.5]undecane (hereinafter, abbreviated as "antioxidant W") was used instead of the antioxidant X in production of the housing. The results are shown in Table 1.

(Example 1-6)

A pre-filled drug package was produced and various evaluations were performed in the same way as in Example 1-1 with the exception that 0.5 parts of the antioxidant W was used instead of the antioxidant X in production of the housing. The results are shown in Table 1.

(Example 1-7)

A pre-filled drug package was produced and various evaluations were performed in the same way as in Example 1-1 with the exception that 0.05 parts of the antioxidant W and 0.15 parts of the light stabilizer Z were used instead of the antioxidant X in production of the housing. The results are shown in Table 1.

(Example 1-8)

A pre-filled drug package was produced and various evaluations were performed in the same way as in Example 1-1 with the exception that polysorbate 80 was added as a non-ionic surfactant such as to have a concentration of 0.04 mg/mL in preparation of the protein solution formulation. The results are shown in Table 1.

(Example 1-9)

A pre-filled drug package was produced and various evaluations were performed in the same way as in Example 1-1 with the exception that a copolymer of a cycloolefin and a chain olefin (copolymer B) that was produced as described below was used instead of the hydrogenated cycloolefin ring-opened polymer (hydrogenated product A) in production of the housing. The results are shown in Table 1.

<<Production of copolymer of cycloolefin and chain olefin (copolymer B)>>

In a stream of nitrogen at normal temperature, norbornene (120 kg) was added into a reactor that had been charged with 258 L of cyclohexane and was stirred for 5 minutes. In addition, triisobutylaluminum was added such that the concentration thereof in the system was 1.0 mL/L. Next, ethylene was circulated at normal pressure while performing stirring in order to convert the system to an ethylene atmosphere. An autoclave internal temperature of 70°C was maintained while raising the internal pressure to a gauge pressure of 6 kg/cm$^2$ with ethylene. After 10 minutes of stirring, 0.4 L of a pre-prepared toluene solution containing isopropylidene(cyclopentadienyl)(indenyl)zirconium dichloride and methylalumoxane was added into the system to initiate a copolymerization reaction of ethylene and norbornene. The catalyst concentration relative to the entire system at this point was 0.018 mmol/L of isopropylidene(cyclopentadienyl)(indenyl)zirconium dichloride and 8.0 mmol/L of methylalumoxane. Ethylene was continuously supplied into the system during the copolymerization reaction in order to maintain the temperature at 70°C and the internal pressure at a gauge pressure of 6 kg/cm$^2$. After 60 minutes, isopropyl alcohol was added to stop the copolymerization reaction. Depressurization was performed, and then a polymer solution was removed and was brought into contact with an aqueous solution of 5 L of concentrated hydrochloric acid added to 1 m$^3$ of water under vigorous stirring in a ratio of 1:1 to cause catalyst residue to move into the aqueous phase. This contacted liquid mixture was left to settle, the aqueous phase was separated and removed, and washing with water was performed twice to purify and separate a polymerization liquid phase.

The polymerization liquid phase that had been purified and separated was then brought into contact with 3 equivalents of acetone under vigorous stirring to cause precipitation of a copolymer. Thereafter, solid (copolymer) was collected by filtration and was thoroughly washed with acetone. The solid was added into acetone such as to have a concentration of 40 kg/m$^3$, and an extraction operation was subsequently performed under conditions of 2 hours at 60°C in order to extract unreacted monomer. After this extraction, solid was collected by filtration and was dried under circulation of nitrogen at 130°C and 350 mmHg for 12 hours to yield an ethylene-norbornene copolymer (copolymer B).

The ethylene-norbornene copolymer (copolymer B) was pelletized in the same way as the hydrogenated product A of Example 1-1. The pelletized ethylene-norbornene copolymer (copolymer B) had a weight-average molecular weight (Mw) of 96,000, a molecular weight distribution (Mw/Mn) of 2.4, and a Tg of 138°C.

(Example 1-10)

A pre-filled drug package was produced and various evaluations were performed in the same way as in Example 1-9 with the exception that polysorbate 80 was added as a non-ionic surfactant such as to have a concentration of 0.04 mg/mL in preparation of the protein solution formulation. The results are shown in Table 1.

(Example 1-11)

A pre-filled drug package was produced and various evaluations were performed in the same way as in Example 1-1 with the exception that pre-drying was not performed in production of the housing. The results are shown in Table 1.

(Example 1-12)

A pre-filled drug package was produced and various evaluations were performed in the same way as in Example 1-8 with the exception that pre-drying was not performed in production of the housing. The results are shown in Table 1.

(Example 1-13)

<Preparation of protein solution formulation>

A protein solution formulation was obtained in the same way as in Example 1-1.

<Production of housing (packaging body)>

<<Production of hydrogenated product A and resin pellets containing hydrogenated product A>>

A hydrogenated cycloolefin ring-opened polymer (hydrogenated product A) and resin pellets containing the hydrogenated product A were obtained in the same way as in Example 1-1.

<<Pre-drying>>

A hot-air dryer was used to dry (pre-dry) the resin pellets obtained as described above in a nitrogen atmosphere under conditions of an atmosphere temperature of 100°C and a drying time of 6 hours. The oxygen concentration of the resin after this pre-drying was measured. The result is shown in Table 1.

<<Shaping>>

Using low-density polyethylene (produced by Japan Polyethylene Corporation; product name: NOVATEC® LD LF640MA (NOVATEC is a registered trademark in Japan, other countries, or both)), a modified polyolefin adhesive resin (produced by Mitsubishi Chemical Corporation; product name: MC719), and the pre-dried resin pellets (hydrogenated product A) obtained as described above as shaping materials, a film melt-extrusion molding machine was used to shape a multilayer film in which a layer formed of low-density polyethylene (thickness: 105 $\mu$m), a layer formed of the modified polyolefin adhesive resin (thickness: 15 $\mu$m), and a layer formed of the resin containing the hydrogenated product A (thickness: 30 $\mu$m) were stacked in this order. This multilayer film was used to measure the water contact angle of a formulation contacting section (surface at side corresponding to layer formed of resin containing hydrogenated product A). The result is shown in Table 1.

Separately to the above, a sealing member including a roughly circular tube shaped stopper and a roughly circular tube shaped outer tube member (external diameter: 5 mm; internal diameter: 3 mm; length: 10 mm) fitting around the outside of the stopper was prepared. A liquid contacting surface of the stopper included in the sealing member was laminated by an ETFE (tetrafluoroethylene-ethylene copolymer) film.

Two roughly rectangular multilayer film pieces were cut out from the multilayer film described above. These multilayer film pieces were overlapped with the outer tube member of the above-described sealing member sandwiched therebetween such that layers formed of the resin containing the hydrogenated product A faced each other, and the perimeters of these multilayer film pieces were welded by heat sealing to obtain a packaging body (width: 115 mm; length: 170 mm). Note that the welding width was 5 mm for edges at both sides and was 3 mm at a narrowest location. Also note that the welding by heat sealing was performed at 255°C for 4 seconds. In addition, a hole for hanging was provided in the packaging body at a position at an opposite side in the length direction thereof relative to the sealing member.

<Production of pre-filled drug package (infusion bag)>

The housing and the protein solution formulation described above were used to produce a pre-filled drug package having the configuration illustrated in FIG. 2 by the following procedure. Note that production of the pre-filled drug package was performed in a sterilized environment.

An internal space of the housing was filled with 100 mL of the protein solution formulation through an internal passage included in the outer tube member of the sealing member. Next, the previously described stopper was inserted into the internal passage of the outer tube member (i.e., a state in which an opening of the housing was hermetically sealed through the sealing member was provided) to thereby obtain a pre-filled drug package that had been filled with the protein solution formulation. The obtained pre-filled drug package was used to evaluate the post-storage concentration of a non-ionic surfactant, the occurrence of production of a decomposition product of a non-ionic surfactant, and the inhibition of protein aggregation. The results are shown in Table 1.

(Example 1-14)

A pre-filled drug package was produced and various evaluations were performed in the same way as in Example 1-13 with the exception that polysorbate 80 was added as a non-ionic surfactant such as to have a concentration of 0.04 mg/mL in preparation of the protein solution formulation. The results are shown in Table 1.

(Example 1-15)

A pre-filled drug package was produced and various evaluations were performed in the same way as in Example 1-13 with the exception that pre-drying was not performed in production of the housing. The results are shown in Table 1.

(Example 2-1)

A pre-filled drug package was produced and various evaluations were performed in the same way as in Example 1-1 with the exception that a protein solution formulation prepared as described below was used. The results are shown in Table 2.

<Preparation of protein solution formulation>

Purified Orencia (abatacept) was adjusted to a concentration of 0.1 mg/mL to obtain a protein solution formulation using phosphate buffered saline (pH: 7.2; NaCl: 200 mM; phosphoric acid: 10 mM).

(Examples 2-2, 2-3, 2-4, 2-5, 2-6, 2-7, 2-9, 2-11, 2-13, and 2-15)

A pre-filled drug package was produced and various evaluations were performed in the same way as in each of Examples 1-2, 1-3, 1-4, 1-5, 1-6, 1-7, 1-9, 1-11, 1-13, and 1-15 with the exception that a protein solution formulation prepared in the same way as in Example 2-1 was used. The results are shown in Table 2.

(Examples 2-8, 2-10, 2-12, and 2-14)

A protein solution formulation was prepared in the same way as in Example 2-1 with the exception that polysorbate 80 was added as a non-ionic surfactant such as to have a concentration of 0.04 mg/mL. A pre-filled drug package was then produced and various evaluations were performed in the same way as in each of Examples 1-8, 1-10, 1-12, and 1-14 with the exception that this protein solution formulation was used. The results are shown in Table 2.

(Comparative Example 1-1)

A pre-filled drug package was produced and various evaluations were performed in the same way as in Example 1-1 with the exception that polysorbate 80 was added as a non-ionic surfactant such as to have a concentration of 0.5 mg/mL in preparation of the protein solution formulation. The results are shown in Table 3.

(Comparative Example 1-2)

A pre-filled drug package was produced and various evaluations were performed in the same way as in Example 1-1 with the exception that a housing produced as described below was used. The results are shown in Table 3.

<Production of housing (vial)>

The surface of a housing (pre-treatment housing) obtained in the same way as in Example 1-1 was coated (surface coated) with a silicon oxide thin film through a PECVD process using a plasma CVD apparatus (produced by Samco Inc.; product name: PD-2201LC). The PECVD process was performed by holding the pre-treatment housing inside a vacuum cassette chamber in a state in which a partial vacuum was not interrupted while supplying a gas containing a linear siloxane precursor, optional oxygen, and optional inert gas.

(Comparative Examples 1-3, 1-4, and 1-5)

A pre-filled drug package was produced and various evaluations were performed in the same way as in each of Example 1-1, Example 1-8, and Comparative Example 1-1 with the exception that a vial made of glass was used as a housing. The results are shown in Table 3.

(Comparative Example 1-6)

A pre-filled drug package was produced and various evaluations were performed in the same way as in Example 1-13 with the exception that polysorbate 80 was added as a non-ionic surfactant such as to have a concentration of 0.5 mg/mL in preparation of the protein solution formulation. The results are shown in Table 3.

(Comparative Example 2-1)

A pre-filled drug package was produced and various evaluations were performed in the same way as in Example 2-1 with the exception that polysorbate 80 was added as a non-ionic surfactant such as to have a concentration of 0.5 mg/mL in preparation of the protein solution formulation. The results are shown in Table 4.

(Comparative Example 2-2)

A pre-filled drug package was produced and various evaluations were performed in the same way as in Example 2-1 with the exception that a housing produced in the same way as in Comparative Example 1-2 was used. The results are shown in Table 4.

(Comparative Examples 2-3, 2-4, and 2-5)

A pre-filled drug package was produced and various evaluations were performed in the same way as in each of Example 2-1, Example 2-8, and Comparative Example 2-1 with the exception that a vial made of glass was used as a housing. The results are shown in Table 4.

(Comparative Example 2-6)

A pre-filled drug package was produced and various evaluations were performed in the same way as in Example 2-13 with the exception that polysorbate 80 was added as a non-ionic surfactant such as to have a concentration of 0.5 mg/mL in preparation of the protein solution formulation. The results are shown in Table 4.

Table 1

| | | | | Example 1-1 | Example 1-2 | Example 1-3 | Example 1-4 | Example 1-5 | Example 1-6 | Example 1-7 | Example 1-8 | Example 1-9 | Example 1-10 | Example 1-11 | Example 1-12 | Example 1-13 | Example 1-14 | Example 1-15 |
|---|---|---|---|---|---|---|---|---|---|---|---|---|---|---|---|---|---|---|
| Pre-filled drug package | Housing | Type | | Vial | Vial | Vial | Vial | Vial | Vial | Vial | Vial | Vial | Vial | Vial | Vial | Infusion Bag | Infusion bag | Infusion bag |
| | | Resin, etc. | Polymer | Hydrogenated product A | Hydrogenated product A | Hydrogenated product A | Hydrogenated product A | Hydrogenated product A | Hydrogenated product A | Hydrogenated product A | Hydrogenated product A | Copolymer B | Copolymer B | Hydrogenated product A | Hydrogenated product A | Hydrogenated product A | Hydrogenated product A | Hydrogenated product A |
| | | | Antioxidant, etc. | X | Y | Z | Y, Z | W | W | W, Z | X | X | X | X | X | X | X | X |
| | | Pre-drying | | Yes | Yes | Yes | Yes | Yes | Yes | Yes | Yes | Yes | Yes | No | No | Yes | Yes | No |
| | | Resin oxygen concentration after pre-drying [mass ppm] | | 1.5 | 1.5 | 1.5 | 1.5 | 1.5 | 1.5 | 1.5 | 1.5 | 1.5 | 1.5 | 21 | 21 | 1.5 | 1.5 | 21 |
| | | Contact angle [°] | | 92 | 91 | 94 | 93 | 91 | 93 | 92 | 92 | 94 | 94 | 87 | 88 | 92 | 92 | 88 |
| | | Surface coating | | No | No | No | No | No | No | No | No | No | No | No | No | No | No | No |
| | Protein solution formulation | Protein | | Humira | Humira | Humira | Humira | Humira | Humira | Humira | Humira | Humira | Humira | Humira | Humira | Humira | Humira | Humira |
| | | Initial concentration of non-ionic surfactant [mg/mL] | | 0.00 | 0.00 | 0.00 | 0.00 | 0.00 | 0.00 | 0.00 | 0.04 | 0.00 | 0.04 | 0.00 | 0.04 | 0.00 | 0.04 | 0.00 |
| Post-storage aggregate concentration [aggregates/mL] | | | | 40 | 40 | 40 | 40 | 40 | 50 | 40 | 30 | 60 | 50 | 200 | 200 | 60 | 40 | 200 |
| Post-storage non-ionic surfactant concentration [mg/mL] | | | | 0.0 | 0.0 | 0.0 | 0.0 | 0.0 | 0.0 | 0.0 | 0.04 | 0.00 | 0.04 | 0.00 | 0.02 | 0.0 | 0.04 | 0.00 |

21

| | Example 1-1 | Example 1-2 | Example 1-3 | Example 1-4 | Example 1-5 | Example 1-6 | Example 1-7 | Example 1-8 | Example 1-9 | Example 1-10 | Example 1-11 | Example 1-12 | Example 1-13 | Example 1-14 | Example 1-15 |
|---|---|---|---|---|---|---|---|---|---|---|---|---|---|---|---|
| Post-storage concentration - Initial concentration (concentration change) [mg/mL] | 0.00 | 0.00 | 0.00 | 0.00 | 0.00 | 0.00 | 0.00 | 0.00 | 0.00 | 0.00 | 0.00 | -0.02 | 0.00 | 0.00 | 0.00 |
| Post-storage production of decomposition product | No | No | No | No | No | No | No | No | No | No | No | No | No | No | No |

Tabel 2

| | | Example 2-1 | Example 2-2 | Example 2-3 | Example 2-4 | Example 2-5 | Example 2-6 | Example 2-7 | Example 2-8 | Example 2-9 | Example 2-10 | Example 2-11 | Example 2-12 | Example 2-13 | Example 2-14 | Example 2-15 |
|---|---|---|---|---|---|---|---|---|---|---|---|---|---|---|---|---|
| drug package | Housing — Type | Vial | Vial | Vial | Vial | Vial | Vial | Vial | Vial | Vial | Vial | Vial | Vial | Infusion bag | Infusion bag | Infusion bag |
| | Resin, etc. — Polymer | Hydrogenated product A | Hydrogenated product A | Hydrogenated product A | Hydrogenated product A | Hydrogenated product A | Hydrogenated product A | Hydrogenated product A | Hydrogenated product A | Copolymer B | Copolymer B | Hydrogenated product A | Hydrogenated product A | Hydrogenated product A | Hydrogenated product A | Hydrogenated product A |
| | Artioxidant, etc. | X | Y | Z | Y, Z | W | W | W, Z | X | X | X | X | X | X | X | X |
| | Pre-drying | Yes | Yes | Yes | Yes | Yes | Yes | Yes | Yes | Yes | Yes | No | No | Yes | Yes | No |
| | Resin oxygen concentration after pre-drying [mass ppm] | 1.5 | 1.5 | 1.5 | 1.5 | 1.5 | 1.5 | 1.5 | 1.5 | 1.5 | 1.5 | 21 | 21 | 1.5 | 1.5 | 21 |
| | Contact angle [°] | 92 | 91 | 94 | 93 | 91 | 93 | 92 | 92 | 94 | 94 | 87 | 88 | 92 | 92 | 88 |
| | Surface coating | No | No | No | No | No | No | No | No | No | No | No | No | No | No | No |
| Protein solution formulation | Protein | Orencia | Orencia | Orencia | Orencia | Orencia | Orencia | Orencia | Orencia | Orencia | Orencia | Orencia | Orencia | Orencia | Orencia | Orencia |
| | Initial concentration of non-ionic surfactant [mg/mL] | 0.00 | 0.00 | 0.00 | 0.00 | 0.00 | 0.00 | 0.00 | 0.04 | 0.00 | 0.04 | 0.00 | 0.04 | 0.00 | 0.04 | 0.00 |
| Post-storage aggregate concentration [aggregates/mL] | | 240 | 230 | 200 | 230 | 220 | 250 | 240 | 200 | 250 | 250 | 600 | 500 | 350 | 300 | 600 |

(continued)

| | Example 2-1 | Example | 2-2 Example 2-3 | Example 2-4 | Example 2-5 | Example 2-6 | Example 2-7 | Example 2-8 | Example 2-9 | Example 2-10 | Example 2-11 | Example 2-12 | Example 2-13 | Example 2-14 | Example 2-15 |
|---|---|---|---|---|---|---|---|---|---|---|---|---|---|---|---|
| Post-storage non-ionic surfactant concentration [mg/mL] | 0.0 | 0.0 | 0.0 | 0.0 | 0.0 | 0.0 | 0.0 | 0.04 | 0.00 | 0.04 | 0.00 | 0.02 | 0.0 | 0.04 | 0.00 |
| Post-storage concentration - Initial concentration (concentration change) [mg/mL] | 0.00 | 0.00 | 0.00 | 0.00 | 0.00 | 0.00 | 0.00 | 0.00 | 0.00 | 0.00 | 0.00 | -0.02 | 0.00 | 0.00 | 0.00 |
| Post-storage production of deconpostion product | No | No | No | No | No | No | No | No | No | No | No | No | No | No | No |

Table 3

| | | | | Comparative Example 1-1 | Comparative Example 1-2 | Comparative Example 1-3 | Comparative Example 1-4 | Comparative Example 1-5 | Comparative Example 1-6 |
|---|---|---|---|---|---|---|---|---|---|
| Pre-filled drug package | Housing | Type | | Vial | Vial | Vial | Vial | Vial | Infusion bag |
| | | Resin, etc. | Polymer | Hydrogenated product A | Hydrogenated product A | Glass | Glass | Glass | Hydrogenated product A |
| | | | Antioxidant, etc. | X | X | | | | X |
| | | Pre-drying | | Yes | Yes | - | - | - | Yes |
| | | Resin oxygen concentration after pre-drying [mass ppm] | | 1.5 | 1.5 | - | - | - | 1.5 |
| | | Contact angle [°] | | 92 | 78 | 65 | 65 | 66 | 92 |
| | | Surface coating | | No | Yes | - | - | - | No |
| | Protein solution formulation | Protein | | Humira | Humira | Humira | Humira | Humira | Humira |
| | | Initial concentration of non-ionic surfactant [mg/mL] | | 0.50 | 0.00 | 0.00 | 0.04 | 0.50 | 0.50 |
| Post-storage aggregate concentration [aggregates/mL] | | | | 30 | 250 | 400 | 300 | 50 | 40 |
| Post-storage non-ionic surfactant concentration [mg/mL] | | | | 0.50 | 0.0 | 0.00 | 0.03 | 0.40 | 0.50 |
| Post-storage concentration - Initial concentration (concentration change) [mg/mL] | | | | 0.00 | 0.00 | 0.00 | -0.01 | -0.10 | 0.00 |
| Post-storage production of decomposition product | | | | Yes | No | No | No | Yes | Yes |

Table 4

| | | | | Comparative Example 2-1 | Comparative Example 2-2 | Comparative Example 2-3 | Comparative Example 2-4 | Comparative Example 2-5 | Comparative Example 2-6 |
|---|---|---|---|---|---|---|---|---|---|
| Pre-filled drug package | Housing | Type | | Vial | Vial | Vial | Vial | Vial | Infusion bag |
| | | Resin, etc. | Polymer | Hydrogenated product A | Hydrogenated product A | Glass | Glass | Glass | Hydrogenated product A |
| | | | Antioxidant, etc. | X | X | | | | X |
| | | Pre-diying | | Yes | Yes | - | - | - | Yes |
| | | Resin oxygen concentration after pre-drying [mass ppm] | | 1.5 | 1.5 | - | - | - | 1.5 |
| | | Contact angle [°] | | 92 | 78 | 65 | 65 | 66 | 92 |
| | | Surface coating | | No | Yes | - | - | - | No |
| | Protein solution formulation | Protein | | Orencia | Orencia | Orencia | Orencia | Orencia | Orencia |
| | | Initial concentration of non-ionic surfactant [mg/mL] | | 0.50 | 0.00 | 0.00 | 0.04 | 0.50 | 0.50 |
| Post-storage aggregate concentration [aggregates/mL] | | | | 200 | 1000 | 4000 | 3500 | 300 | 300 |
| Post-storage non-ionic surfactant concentration [mg/mL] | | | | 0.50 | 0.0 | 0.00 | 0.03 | 0.40 | 0.50 |
| Post-storage concentration - Initial concentration (concentration change) [mg/mL] | | | | 0.00 | 0.00 | 0.00 | -0.01 | -0.10 | 0.00 |
| Post-storage production of decomposition product | | | | Yes | No | No | No | Yes | Yes |

It can be seen from Tables 1 and 2 that the pre-filled drug packages of Examples 1-1 to 1-15 and Examples 2-1 to 2-15 could inhibit protein aggregation after long-term storage even when the protein solution formulation had a low non-ionic surfactant concentration of not less than 0 mg/mL and less than 0.05 mg/mL.

On the other hand, it can be seen from Table 3 that production of a decomposition product of a non-ionic surfactant after long-term storage occurred in Comparative Examples 1-1, 1-5, and 1-6 in which the protein solution formulation had a high non-ionic surfactant concentration (0.50 mg/mL).

It can also be seen from Table 3 that in Comparative Example 1-2 in which a formulation contacting section was not formed of a specific resin as a result of surface coating being performed and in Comparative Examples 1-3 and 1-4 in which a housing made of glass was used, it was not possible to inhibit protein aggregation after long-term storage compared to Examples 1-1 to 1-15 in which the same protein was used.

Moreover, it can be seen from Table 4 that production of a decomposition product of a non-ionic surfactant after long-term storage occurred in Comparative Examples 2-1, 2-5, and 2-6 in which the protein solution formulation had a high non-ionic surfactant concentration (0.50 mg/mL).

It can also be seen from Table 4 that in Comparative Example 2-2 in which a formulation contacting section was not formed of a specific resin as a result of surface coating being performed and in Comparative Examples 2-3 and 2-4 in which a housing made of glass was used, it was not possible to inhibit protein aggregation after long-term storage compared to Examples 2-1 to 2-15 in which the same protein was used.

INDUSTRIAL APPLICABILITY

According to the present disclosure, it is possible to provide a pre-filled drug package that can inhibit protein aggregation after long-term storage even when the pre-filled drug package accommodates a protein solution formulation having a low non-ionic surfactant concentration.

Moreover, according to the present disclosure, it is possible to provide a method of producing a pre-filled drug package that can inhibit protein aggregation after long-term storage even when the pre-filled drug package accommodates a protein solution formulation having a low non-ionic surfactant concentration.

REFERENCE SIGNS LIST

| | |
|---|---|
| 10 | vial (pre-filled drug package) |
| 11 | vessel (housing) |
| 12 | cap (sealing member) |
| 13, 23 | protein solution formulation |
| 20 | infusion bag (pre-filled drug package) |
| 21 | packaging body (housing) |
| 22 | port (sealing member) |
| 24 | hole |

**Claims**

1.  A pre-filled drug package comprising: a protein solution formulation; and a housing accommodating the protein solution formulation, wherein

    the protein solution formulation has a non-ionic surfactant concentration of not less than 0 mg/mL and less than 0.05 mg/mL, and
    at least part of a section of the housing that is in contact with the protein solution formulation is formed of a resin containing either or both of a hydrogenated cycloolefin ring-opened polymer and a copolymer of a cycloolefin and a chain olefin.

2.  The pre-filled drug package according to claim 1, wherein the section of the housing that is in contact with the protein solution formulation has a water contact angle of 90° or more.

3.  The pre-filled drug package according to claim 1 or 2, further comprising a sealing member, wherein the housing has an opening, and the opening is hermetically sealed by the sealing member.

4. The pre-filled drug package according to any one of claims 1 to 3, wherein the protein solution formulation contains either or both of an antibody and an antigen binding fragment of the antibody.

5. The pre-filled drug package according to claim 4, wherein the antibody is at least one selected from the group consisting of chimeric antibodies, human antibodies, humanized antibodies, and domain antibodies of any thereof.

6. The pre-filled drug package according to any one of claims 1 to 3, wherein the protein solution formulation contains at least one selected from the group consisting of ofatumumab, cetuximab, tocilizumab, bevacizumab, canakinumab, golimumab, ustekinumab, eculizumab, omalizumab, trastuzumab, pertuzumab, adalimumab, denosumab, mogamulizumab, rituximab, ranibizumab, infliximab, aflibercept, abatacept, etanercept, gemtuzumab ozogamicin, panitumumab, basiliximab, certolizumab pegol, and palivizumab.

7. The pre-filled drug package according to any one of claims 1 to 6, wherein the pre-filled drug package is a vial, an infusion bag, a pre-filled cartridge, an ampoule, a bottle, a pouch, or a blister pack.

8. A method of producing a pre-filled drug package that includes a protein solution formulation and a housing accommodating the protein solution formulation, comprising a step of filling the protein solution formulation, with a nonionic surfactant concentration of not less than 0 mg/mL and less than 0.05 mg/mL, into the housing to obtain the pre-filled drug package, wherein
at least part of a section of the housing that is in contact with the protein solution formulation is formed of a resin containing either or both of a hydrogenated cycloolefin ring-opened polymer and a copolymer of a cycloolefin and a chain olefin.

9. The method of producing a pre-filled drug package according to claim 8, further comprising, in advance of the step of obtaining the pre-filled drug package:

a step of pre-drying the resin; and
a step of shaping the resin after the pre-drying to obtain the housing.

10. The method of producing a pre-filled drug package according to claim 9, wherein the resin has an oxygen concentration of 10 mass ppm or less after the pre-drying.

11. The method of producing a pre-filled drug package according to claim 9 or 10, wherein the pre-drying is performed in an inert gas atmosphere.

12. The method of producing a pre-filled drug package according to any one of claims 9 to 11, wherein the pre-drying has a drying temperature of not lower than 80°C and not higher than 120°C.

13. The method of producing a pre-filled drug package according to any one of claims 8 to 12, wherein the protein solution formulation contains either or both of an antibody and an antigen binding fragment of the antibody.

14. The method of producing a pre-filled drug package according to claim 13, wherein the antibody is at least one selected from the group consisting of chimeric antibodies, human antibodies, humanized antibodies, and domain antibodies of any thereof.

15. The method of producing a pre-filled drug package according to any one of claims 8 to 12, wherein the protein solution formulation contains at least one selected from the group consisting of ofatumumab, cetuximab, tocilizumab, bevacizumab, canakinumab, golimumab, ustekinumab, eculizumab, omalizumab, trastuzumab, pertuzumab, adalimumab, denosumab, mogamulizumab, rituximab, ranibizumab, infliximab, aflibercept, abatacept, etanercept, gemtuzumab ozogamicin, panitumumab, basiliximab, certolizumab pegol, and palivizumab.

16. The method of producing a pre-filled drug package according to any one of claims 8 to 15, wherein the method is a method of producing a vial, an infusion bag, a pre-filled cartridge, an ampoule, a bottle, a pouch, or a blister pack.

# FIG. 1

# FIG. 2

## INTERNATIONAL SEARCH REPORT

| International application No. |
|---|
| PCT/JP2021/002477 |

**A. CLASSIFICATION OF SUBJECT MATTER**
A61J 1/05(2006.01)i
FI: A61J1/05 311

According to International Patent Classification (IPC) or to both national classification and IPC

**B. FIELDS SEARCHED**

Minimum documentation searched (classification system followed by classification symbols)
A61J1/05

Documentation searched other than minimum documentation to the extent that such documents are included in the fields searched
Published examined utility model applications of Japan       1922–1996
Published unexamined utility model applications of Japan     1971–2021
Registered utility model specifications of Japan             1996–2021
Published registered utility model applications of Japan     1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | WO 01/17542 A1 (CHUGAI PHARMACEUTICAL CO., LTD.) 15 March 2001 (2001-03-15) page 4, line 16 to page 5, line 3, page 7, lines 5-13, page 11, lines 16-20 | 1–16 |
| Y | JP 2018-535974 A (SIO2 MEDICAL PRODUCTS, INC.) 06 December 2018 (2018-12-06) paragraph [0289] | 1–16 |
| Y | JP 2008-93830 A (JSR CORPORATION) 24 April 2008 (2008-04-24) paragraph [0050] | 9–16 |

☐ Further documents are listed in the continuation of Box C.       ☒ See patent family annex.

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| | |
|---|---|
| "T" | later document published after the international filing date or priority date and not in conflict with the application but cited to understand the principle or theory underlying the invention |
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 16 March 2021 (16.03.2021) | 23 March 2021 (23.03.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**
Information on patent family members

International application No.

PCT/JP2021/002477

| Patent Documents referred in the Report | Publication Date | Patent Family | Publication Date |
|---|---|---|---|
| WO 01/17542 A1 | 15 Mar. 2001 | US 7253142 B1 column 3, lines 25-49, column 4, line 66 to column 5, line 13, column 7, lines 45-51 EP 1232753 A1 | |
| JP 2018-535974 A | 06 Dec. 2018 | US 2018/0325728 A1 paragraph [0361] WO 2017/087871 A1 CA 3005132 A1 KR 10-2018-0105123 A CN 108883057 A | |
| JP 2008-93830 A | 24 Apr. 2008 | KR 10-2008-0031803 A | |

Form PCT/ISA/210 (patent family annex) (January 2015)

**EP 4 098 238 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018537170 A

- JP 2016155327 A